# EUROPÄISCHE PATENTSCHRIFT

(11) **EP 1 242 351 B1**
(45) Veröffentlichungstag und Bekanntmachung des Hinweises auf die Patenterteilung: **28.04.2004**
(21) Anmeldenummer: 00981236.3
(22) Anmeldetag: 04.11.2000
(51) Int. Cl.: C07C 45/00

(54) **VERFAHREN ZUR HYDROFORMYLIERUNG OLEFINISCH UNGESÄTTIGTER VERBINDUNGEN**
METHOD FOR HYDROFORMYLATING OLEFINICALLY UNSATURATED COMPOUNDS
PROCEDE D'HYDROFORMULATION DE COMPOSES OLEFINIQUEMENT INSATURES

(30) Priorität: 13.11.1999 DE 19954665
(43) Veröffentlichungstag der Anmeldung: 25.09.2002
(73) Patentinhaber: Celanese Chemicals Europe GmbH, 60439 Frankfurt am Main (DE)
(72) Erfinder: HÖFS, Wolfgang, 46147 Oberhausen (DE)
(86) Internationale Anmeldenummer: PCT/EP2000/010881
(87) Internationale Veröffentlichungsnummer: WO 2001/036361

(56) Entgegenhaltungen:
- DE-A- 2 627 354

## Beschreibung

Die Erfindung betrifft ein verbessertes Verfahren zur Hydroformylierung olefinisch ungesättigter Verbindungen in Gegenwart einer wässrigen, wasserlösliche Rhodium-Komplexverbindungen enthaltenden Katalysatorlösung durch Verwendung einer Reaktionskolonne als Reaktor.

Die Reaktion von Verbindungen, die olefinische Doppelbindungen enthalten, mit Kohlenmonoxid und Wasserstoff, ist das gängige technische Verfahren zur Herstellung von Aldehyden (Oxosynthese).

Der Prozeß ist nicht auf den Einsatz olefinischer Kohlenwasserstoffe beschränkt, sondern erstreckt sich auch auf Ausgangsstoffe, die ausser der Doppelbindung noch funktionelle Gruppen aufweisen, vorwiegend solche, die unter den Reaktionsbedingungen unverändert bleiben.

Die klassische Oxosynthese arbeitet mit Kobalt als Katalysator. Seine Wirksamkeit beruht auf der Bildung von Kobaltcarbonylverbindungen unter der Einwirkung von Wasserstoff und Kohlenmonoxid bei Drücken oberhalb 20 MPa und Temperaturen von etwa 120°C und mehr auf metallisches Kobalt oder Kobaltverbindungen.

In den letzten 30 Jahren wurde Kobalt zunehmend durch Rhodium als Katalysator ersetzt. Das Platinmetall wird als Komplexverbindung eingesetzt, die neben Kohlenmonoxid vorzugsweise Phosphine als Liganden enthält. Rhodium als Katalysator erlaubt es, bei niedrigeren Drücken zu arbeiten, überdies erzielt man höhere Ausbeuten und bevorzugt werden die für die Weiterverarbeitung wertvolleren unverzweigten Produkte gebildet, wenn man von geradkettigen endständigen Olefinen ausgeht.

Eine weitere Vervollkommnung der Oxosynthese bedeutet der Übergang von homogen im Reaktionsmedium, d.h. im Einsatzmaterial und im Reaktionsprodukt gelösten Katalysatoren zu wässrigen Katalysatorlösungen, die als eigene Phase getrennt von Einsatzstoff und Umsetzungsprodukt vorliegen. Diese Variante der Reaktion ist z.B. in der DE-B-26 27 354 beschrieben. Ihr besonderer Vorteil ist die leichte Trennung von Reaktionsprodukt und Katalysator, die schonend, ohne Anwendung thermischer Verfahrensschritte, erfolgt und daher Verluste vermeidet, die durch Folgereaktionen der entstandenen Aldehyde eintreten. Weiterhin erzielt man sehr hohe Ausbeuten und bei Verwendung unverzweigter endständiger Olefine erhält man ganz überwiegend n-Aldehyde.

Die praktische Durchführung der Oxosynthese mit wässsriger Katalysatorphase erfolgt üblicherweise in Rührreaktoren, in denen das in Wasser gelöste Katalysatorsystem vorgelegt ist. Olefinisch ungesättigte Verbindungen und Synthesegas werden dem Reaktionsgefäss zugeführt und unter inniger Vermischung miteinander umgesetzt. Das Reaktionsprodukt verlässt den Reaktor zusammen mit wässriger Katalysatorlösung, nicht verbrauchten Einsatzstoffen (Synthesegas, Olefin) und Hydrierungsprodukten der olefinisch ungesättigten Verbindungen über ein Tauchrohr. Die Gasphase, im wesentlichen Synthesegas, Olefin und aus dem Olefin gebildeter gesättigter Kohlenwasserstoff, wird in einem Trenngefäss von den Flüssigprodukten getrennt und im Kreislauf wieder dem Reaktor zugeführt. Eine Teilmenge des Kreislaufgases befreit man in einem Kühler von den kondensierbaren Reaktionsprodukten und schleust es in das Abgassystem.

Die im Trenngefäss abgeschiedene Flüssigkeit wird einem Phasentrenner zugeführt. Hier trennt sich das rohe organische Reaktionsprodukt von der wässrigen Katalysatorphase. Während das organische Reaktionsprodukt über eine Pumpe einer Strippkolonne zugeführt wird, fördert eine weitere Pumpe die wässrige Katalysatorphase in den Reaktor zurück, wobei in einem Wärmeaustauscher die Wärme der exothermen Reaktion unter Erzeugung von Prozessdampf abgegeben wird. Zusammen mit der gekühlten Katalysatorlösung kann dem Reaktor Wasser zugeführt werden, um Wasserverluste, die über das Abgas und über das Oxoprodukt auftreten, zu kompensieren. Das in die Strippkolonne eingeleitete Oxorohprodukt führt man im Gegenstrom zu einer Teilmenge des Synthesegases, das sich hierbei mit dem im Rohprodukt gelösten Olefin belädt. Das vorerwärmte Synthesegas/Olefingemisch wird dem Reaktor zugeführt. Ein weiterer Synthesegasteilstrom wird in einem Wärmetauscher mit Prozesswärme vorerhitzt. Auch das Frischolefin wird vor Eintritt in den Reaktor mit Abwärme der Aldehyddestillation in einem Wärmetauscher vorgewärmt und verdampft, während das Oxorohprodukt aus der Strippkolonne ungekühlt direkt der Destillation zugeführt wird. Für den Fall einer Störung in der Anlage ist schliesslich noch ein Pufferbehälter zur Produktzwischenlagerung vorgesehen.

Nach einer technisch besonders zweckmässigen Ausführungsform einer Produktionsanlage zur Durchführung der Oxosynthese leitet man Synthesegas und Olefin über Doppelbrausen, die als Vorverteiler dienen, in den, wässrige Katalysatorlösung enthaltenden, Reaktor. Die Feinverteilung der Reaktanten im Reaktionsgemisch erfolgt über einen Begasungsrührer. Zur Ableitung der Reaktionswärme ist der Reaktor mit einem Kühlregister versehen. Die flüssigen und gasförmigen Komponenten steigen im Reaktor in einem Führungsrohr auf und trennen sich an dessen oberem Ende. Das Gas wird entweder in den Reaktor zurückgeführt oder als Abgas aus dem Reaktionssystem abgeleitet. Die wässrige Katalysatorlösung trennt sich von dem rohen, organischen Produkt. Das Rohprodukt wird einer Strippkolonne aufgegeben, durch das im Gegenstrom geführte Synthesegas von gelöstem Olefin befreit und schliesslich in einer Kolonne in seine Komponenten zerlegt. Die für die Destillation erforderliche Wärme wird direkt über das Kühlregister gewonnen. Hierzu leitet man den flüssigen Aldehyd aus dem Sumpf der Kolonne über einen Phasentrenner in das Kühlregister ein, in dem er verdampft und führt ihn über den Phasentrenner in Dampfform wieder der Kolonne zu.

Die vorstehend beschriebene apparative Realisierung der Oxosynthese mit wässriger Katalysatorphase hat sich in der industriellen Praxis ausgezeichnet bewährt. Dennoch besteht ein Interesse daran, das Verfahren weiter zu optimieren. Diese Aufgabe liegt der vorliegenden Erfindung zugrunde. Im einzelnen gilt das für die Verbesserung der Wirtschaftlichkeit durch Änderung des Verfahrensablaufs und/oder durch Vereinfachung der apparativen Ausgestaltung des Prozesses. Auch die Steigerung des Umsatzes, der Wertproduktausbeute und der Erhöhung der Sicherheit zählen hierzu.

Die Erfindung besteht in einem Verfahren zur Hydroformylierung olefinisch ungesättigter Verbindungen in einem heterogenen Reaktionssystem unter Verwendung einer wässrigen Lösung als Katalysator, die Komplexverbindungen des Rhodiums mit wasserlöslichen organischen Phosphor(III)-verbindungen als Liganden und gegebenenfalls noch überschüssige wasserlösliche organische Phosphor(III)-verbindungen enthält, bei Drücken von 0,4 bis 10 MPa und Temperaturen von 50 bis 180°C. Es ist dadurch gekennzeichnet, dass die Umsetzung der Reaktionspartner in einer Reaktionskolonne erfolgt.

Neben der Durchführung der Reaktion zwischen olefinischer Verbindung und Synthesegas in einem heterogenen Reaktionssystem mit wässriger Katalysatorphase ist ein wesentliches Merkmal der Erfindung, dass die Umsetzung in einer Reaktionskolonne als Reaktor erfolgt.

Die Unterschiede des neuen Verfahrens gegenüber den bisher gebräuchlichen Prozessen mit einem Rührkessel als zentralem Apparat der Reaktionsanlage sind augenfällig. Abgesehen von weiteren Änderungen sind die Durchmischung der Reaktionspartner und der Katalysatorlösung ohne Verwendung eines Rührers sowie das Fehlen einer separaten Strippvorrichtung zur Rückgewinnung des im Produkt gelösten Olefins von besonderer Bedeutung.

Unter dem Begriff Reaktionskolonnen werden die in der chemischen Technik insbesondere zur Destillation, Rektifikation und Extraktion verwendeten Apparate verstanden. Sie sind als Hydroformylierungsreaktor mit Einspeiseöffnungen für die Reaktionspartner und die Katalysatorlösung und mit Entnahmevorrichtungen für Produkt, Katalysatorlösung und Abgas versehen. Als Reaktionskolonnen können die in der industriellen Praxis vielfach genutzten, unterschiedlichen Kolonnentypen eingesetzt werden, insbesondere Bodenkolonnen, Packungskolonnen und Füllkörperkolonnen. In ihnen erfolgt die Zuführung der Katalysatorlösung am Kolonnenkopf, zweckmässig auf dem obersten Kolonnenboden. Unterhalb des Kolonnenkopfes, vorzugsweise im oberen Bereich (d.h. in der oberen Hälfte) der Kolonne, wird die olefinische Verbindung in den Reaktor eingespeist. Katalysatorlösung, Olefin und Reaktionsprodukt, die sich im Kolonnenraum abwärts bewegen, strömt das, am Kolonnenfuß eingeführte, Synthesegas entgegen. Die Entnahme von Reaktionsprodukt und Katalysatorlösung erfolgt im unteren Teil (der unteren Hälfte) des Reaktors, die gasförmigen Anteile entweichen im oberen Bereich der Kolonne.

Die Zuleitung von Reaktionspartnem und Katalysatorlösung zum Reaktor ist nicht auf jeweils eine Einspeisestelle beschränkt. Es ist also nicht erforderlich, dass die Gesamtmenge Olefin ausschliesslich an einer Stelle und im oberen Bereich und die Gesamtmenge Synthesegas ausschliesslich am Fuss der Reaktionskolonne in den Reaktionsraum einströmen. Vielmehr können die Reaktanten an unterschiedlichen Orten entlang der Kolonne zugeführt werden. Hierbei kann die Anzahl der Einspeisestellen maximal gleich der Anzahl der Trennstufen der Kolonne sein. Durch Ausstattung des Reaktors mit mehreren Zuleitungen für die einzelnen Reaktionsteilnehmer lässt er sich den Erfordernissen der jeweiligen Reaktion, z.B. hinsichtlich der Reaktivität des eingesetzten Olefins, der angestrebten Reaktionsgeschwindigkeit, der Vollständigkeit des Umsatzes und der gewünschten Zusammensetzung des Reaktionsproduktes, individuell anpassen. Lediglich als Beispiel sei die Reaktionsfähigkeit des Olefins näher betrachtet. Bei schnell reagierenden Olefinen wird man dem Reaktor eine grössere Menge Olefin zuführen können als bei reaktionsträgen Olefinen mit der Folge, dass man die reaktiven, ungesättigten Kohlenwasserstoffe dem Reaktor nicht nur über eine, sondern über mehrere Zuleitungen aufgibt. Diese Möglichkeit, die Mengenströme zu variieren, erlaubt es überdies, den gleichen Reaktor für die Umsetzung verschiedener Einsatzmaterialien zu verwenden. Die Zufuhr von Katalysatorlösung an verschiedenen Stellen des Reaktors wird sich auf Sonderfälle beschränken, z.B. zur Aufrechterhaltung der erforderlichen Katalysatorkonzentration bei erhöhtem, durch Verunreinigungen in den Ausgangsstoffen bedingtem, Katalysatorverbrauch oder auch zur Steuerung der Temperatur durch Abführung von Reaktionswärme aus dem Reaktor. Unterhalb des Kolonnenkopfes ist es möglich, die Katalysatorlösung entweder vom Olefin getrennt oder auch mit diesem gemeinsam der Kolonne zuzuführen.

Eine Reaktionskolonne statt eines Rührkessels als Reaktor einzusetzten, erweist sich auch deshalb als vorteilhaft, weil in der Kolonne mehrere Trennstufen (z.B. Kolonnenböden) hintereinander geschaltet sind. Die Hintereinanderschaltung mehrerer Trennstufen in der Kolonne bewirkt, dass die Charakteristik des Rührkessels in den einer Rührkesselkaskade überführt wird. Olefin und Synthesegas reagieren in Gegenwart der Katalysatorlösung miteinander bis zur Einstellung des jeweiligen chemischen und physikalischen Gleichgewichts. Daher ist der Umsatz in der Reaktionskolonne höher als in einem Rührkessel. Bewährt haben sich im erfindungsgemäßen Verfahren Reaktionskolonnen mit 5 bis 120, insbesondere 15 bis 40, Trennstufen.

Für die Auswahl der im Einzelfall geeigneten Reaktionskolonne ist ihr Belastungsbereich massgebend. Unter diesem Begriff versteht man die gleichmässige strömungstechnische Beanspruchung des Kolonnenquerschnitts durch den aufsteigenden Gas-/Massestrom und den rücklaufenden Flüssigkeits-/Massestrom. Jede Reaktionskolonne hat einen bestimmten, von der Art ihrer Einbauten und den Eigenschaften des Einsatzgemisches abhängigen Belastungsbereich, innerhalb dessen eine gleichmässige Strömungsbelastung und damit der Wärme- und Stofftransport gesichert sind.

Die Hydroformylierungsreaktion ist stark exotherm. Die bei der Umsetzung entwickelte Wärme kann entweder reaktionsintem zur Vorerwärmung der Reaktionspartner und zur Destillation des Reaktionsproduktes und/oder extem zur Erzeugung von Dampf verwendet werden. Die Abführung der thermischen Energie erfolgt zweckmässig über das Reaktionsprodukt und die im Kreis geführte Katalysatorlösung, doch ist die Wärmegewinnung nicht auf diese Ausführungsformen begrenzt.

Das in der Reaktionskolonne aufsteigende Synthesegas strömt, wie schon gesagt, dem Reaktionsprodukt entgegen. Die gasförmige Phase durchsetzt den Flüssigkeitsstrom, extrahiert dabei im Reaktionsprodukt gelöstes, nicht umgesetztes Olefin und transportiert es in die Reaktionszone. Auf diese Weise wird der Olefinumsatz deutlich verbessert, ohne dass in der Syntheseanlage eine spezielle Extraktionseinheit installiert werden muß.

Als Katalysatoren werden wasserlösliche Rhodium-Komplexverbindungen eingesetzt, die wasserlösliche Phosphor(III)-verbindugnen als Liganden enthalten. Beispiele für wasserlösliche Phosphor(III)-verbindungen, die mit Rhodium Komplexverbindungen bilden, sind Triarylphosphine, Trialkylphosphine und arylierte bzw. alkylierte Diphosphine, deren organische Reste Sulfonsäuregruppen oder Carboxylgruppen enthalten. Ihre Herstellung und Anwendung ist z.B. aus DE-B-26 27 354, EP 0 103 810 B1, EP 0 163 234 B1 und EP 0 571 819 A1 bekannt. Weitere Gruppen geeigneter Verbindungen sind sulfonierte oder carboxylierte organische Phosphite sowie heterocyclische Verbindungen des dreibindigen Phosphors.

Die Bedingungen, unter denen die Umsetzung abläuft, können innerhalb weiter Grenzen variiert und den individuellen Gegebenheiten angepasst werden. Sie hängen u.a. vom Einsatzmaterial, vom ausgewählten Katalysatorsystem und vom angestrebten Umsetzungsgrad ab. Üblicherweise führt man die Hydroformylierung der Einsatzstoffe bei Temperaturen von 50 bis 180°C durch. Bevorzugt hält man Temperaturen von 80 bis 140°C und insbesondere von 100 bis 130°C ein. Der Gesamtdruck erstreckt sich über einen Bereich von 0,4 bis 10 MPa, vorzugsweise 1 bis 6 MPa und insbesondere 1,5 bis 5 MPa. Das molare Verhältnis von Wasserstoff zu Kohlenmonoxid bewegt sich üblicherweise zwischen 1:10 bis 10:1, Mischungen, die Wasserstoff und Kohlenmonoxid im molaren Verhältnis 3:1 bis 1:3 und insbesondere 1:1 enthalten, werden bevorzugt.

Die Rhodium-Konzentration beträgt 20 bis 1000 Gew.-ppm, vorzugsweise 50 bis 500 Gew.-ppm und insbesondere 100 bis 300 Gew.-ppm, jeweils bezogen auf die wässrige Katalysatorlösung. Obgleich es möglich ist, als Katalysator die stöchiometrisch zusammengesetzte Rhodium-Phosphor-Komplexverbindung einzusetzen, arbeitet man bevorzugt in Gegenwart von überschüssigem Phosphorliganden, d.h. Ligand, der mit Rhodium keine komplexe Bindung eingegangen ist. Je mol Rhodium wendet man vorzugsweise 3 bis 200 mol Phosphor in Form einer wasserlöslichen organischen Phosphorverbindung an. Besonders bewährt haben sich molare Verhältnisse von Rhodium zu Phosphor im Bereich 1:50 bis 1:100. Der Rhodium-Phosphor-Komplexkatalysator braucht nicht einheitlich zusammengesetzt zu sein, sondern kann z.B. aus einem Gemisch von Rhodium-Komplexverbindungen bestehen, die sich durch die Art der Phosphorliganden unterscheiden. Ebenso kann der in der wässrigen Katalysatorlösung enthaltene Phosphorligand aus einem Gemisch unterschiedlicher wasserlöslicher organischer Phosphorverbindungen zusammengesetzt sein. Man bildet den Katalysator üblicherweise aus den Komponenten Rhodium oder Rhodiumverbindung, organische Phosphorverbindung und Synthesegas unter den Bedingungen der Hydroformylierungsreaktion. Er kann der Reaktionsstufe aber auch präformiert, d.h. gesondert hergestellt, zugeführt werden. Es hat sich als zweckmässig erwiesen, den Katalysator im Kreis zu führen und gegebenenfalls auftretende Katalysatorverluste durch Zufuhr von Frischkatalysator auszugleichen.

Um den Umsatz je Zeiteinheit von olefinsich ungesättigten Verbindungen, die in der wässrigen Katalysatorlösung nur wenig löslich sind, zu erhöhen, kann es sich empfehlen, dieser Lösung ein Phasentransferreagenz (Lösungsvermittler) zuzusetzen. Es verändert die physikalischen Eigenschaften der Grenzflächen zwischen den beiden flüssigen Phasen und erleichtert den Übergang des organischen Reaktanten in die wässrige Katalysatorphase. Lösungsvermittelnd wirken Verbindungen, deren hydrophile Gruppen ionisch (anionisch oder kationisch) oder nichtionisch sind. Beispiele für anionenaktive Verbindungen sind u.a. die Natrium-, Kalium- oder Ammoniumsalze von Carbonsäuren, vorzugsweise solchen mit 8 bis 20 Kohlenstoffatomen. Zu den kationischen Lösungsvermittlern zählen Tetraalkylammonium- und N-Alkylpyridiniumsalze. Nichtionische Phasentransferreagenzien sind z.B. Alkyl- und Alkylphenylpolyethylenglykole und Trialkylaminoxide.

Die Reaktionspartner Olefin und Synthesegas können dem Reaktor vorerhitzt zugeführt werden. Die flüssige Phase verlässt die Reaktionskolonne im unteren Teil, vorzugsweise am Kolonnenfuss und gelangt über einen Wärmeaustauscher in einen Phasentrenner. Hier erfolgt die Zerlegung in das organische Reaktionsprodukt und die wässrige Katalysatorlösung. Das dem Reaktor im oberen Teil, insbesondere am Kolonnenkopf entnommene Abgas besteht im wesentlichen aus Kohlenmonoxid und Wasserstoff. Daneben kann es Reaktionsprodukt, durch Hydrierung der olefinischen Verbindungen entstandene gesättigte Verbindungen und Wasser enthalten. Olefinisches Einsatzmaterial findet sich im Abgas insbesondere dann, wenn z.B. aus Gründen der Prozessökonomie die Umsetzung nicht bis zum völligen Verbrauch der ungesättigten Verbindung durchgeführt wurde. Das Olefin kann aus dem Abgas nach bekannten Verfahren, z.B. durch Auskondensieren, zurückgewonnen oder in einer zweiten Reaktionsstufe zu Hydroformylierungsprodukt umgesetzt werden. Auch Reaktionsprodukt, das im Abgas enthalten ist, wird abgetrennt und in die Reaktionskolonne zurückgeführt oder unmittelbar in die Destillationsstufe geleitet.

Das erfindungsgemässe Verfahren kann auf olefinisch ungesättigte Verbindungen beliebiger Struktur angewandt werden. Dementsprechend sind als Ausgangsmaterial geeignet sowohl Olefine mit innenständiger als auch mit endständiger Doppelbindung und ebenso geradkettige oder verzweigte Olefine. Überdies können die Olefine auch noch durch funktionelle Gruppen substituiert sein, insbesondere solche, die im Verlauf der Reaktion nicht verändert werden.

Auch mehrfach olefinisch ungesättigte Verbindungen kommen als Einsatzstoffe in Betracht. Besonders bewährt hat sich das Verfahren bei der Hydroformylierung olefinisch ungesättigter Kohlenwasserstoffe mit 3 bis 12 Kohlenstoffatomen im Molekül, vorzugsweise Propylen und die isomeren Butene.

In den beigefügten Zeichnungen werden beispielhaft mögliche Ausführungsformen des erfindungsgemässen Verfahrens schematisch dargestellt.

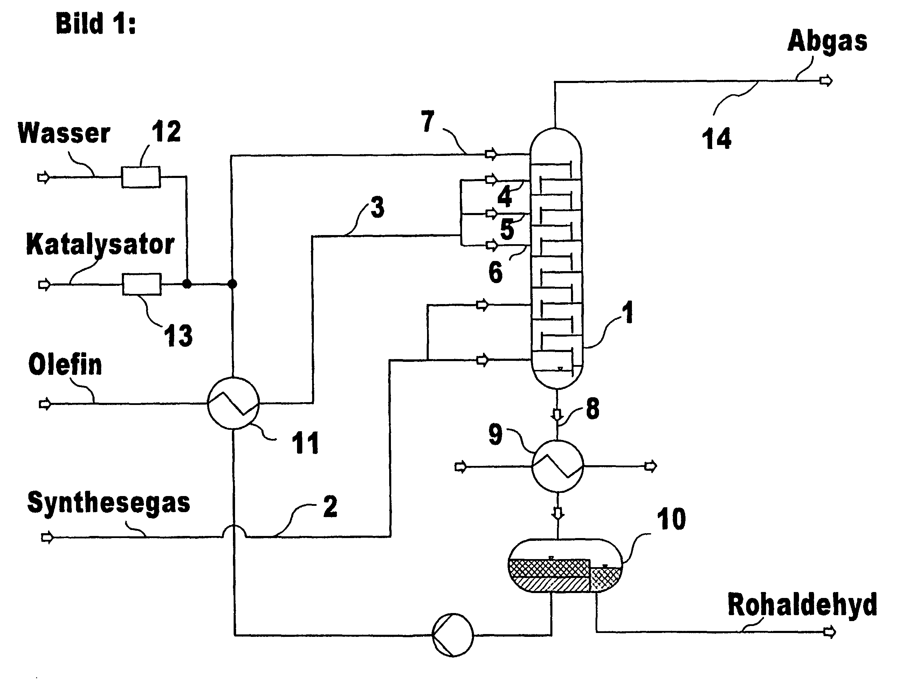

Nach dem in Bild 1 skizzierten Verfahrensablauf werden einer Reaktionskolonne 1 am Kolonnenfuss über eine Leitung 2 Synthesegas und über eine Leitung 3 an Einspeisestellen 4, 5 und 6, die an der Längsseite des Reaktors angeordnet sind, Olefin zugeführt. Die Katalysatorlösung wird über eine Leitung 7 am Kolonnenkopf auf die oberste Trennstufe der Kolonne gepumpt. In Gegenwart des Katalysators reagieren im Reaktor nach unten strömendes Olefin mit aufsteigendem Synthesegas bis zur Gleichgewichtseinstellung zu Aldehyd. Im Produkt gelöstes Olefin wird durch den nach oben gerichteten Synthesegasstrom aus der flüssigen Phase abgetrennt und ebenfalls zu Aldehyd umgesetzt. Der von Olefin befreite Rohaldehyd wird zusammen mit der Katalysatorlösung der Reaktionskolonne im unteren Teil entnommen und unmittelbar über eine Leitung 8 einem Wärmeaustauscher 9 zugeleitet. Die gewonnene Wärme kann man z.B. zur Erzeugung von Wasserdampf und/oder zur Destillation des Reaktionsproduktes verwenden. Dem Wärmeaustauscher schliesst sich ein Separator 10 an, in dem organisches Produkt und wässrige Katalysatorlösung getrennt werden. Die Katalysatorphase wird nach Abgabe von Restwärme in einem Wärmeaustauscher 11 auf den Kopf der Reaktionskolonne 1 gepumpt, gegebenenfalls nach Zugabe von Ergänzungsmengen Wasser oder Katalysatorlösung aus Behältern 12 und 13. Über eine Leitung 14 wird dem Reaktionssystem Abgas entnommen. Es enthält überwiegend Kohlenmonoxid und Wasserstoff und in geringen Anteilen u.a. nicht umgesetztes Olefin und Reaktionsprodukt. Olefin und Produkt werden nicht verworfen, sondern können, ebenso wie Kohlenmonoxid und Wasserstoff, noch genutzt werden, beispielsweise im Prozess selbst. Es ist auch möglich, das Gasgemisch mit dem aus dem Abgas gewonnenen Olefin in einem Nachreaktor in weiteres Wertprodukt umzuwandeln.

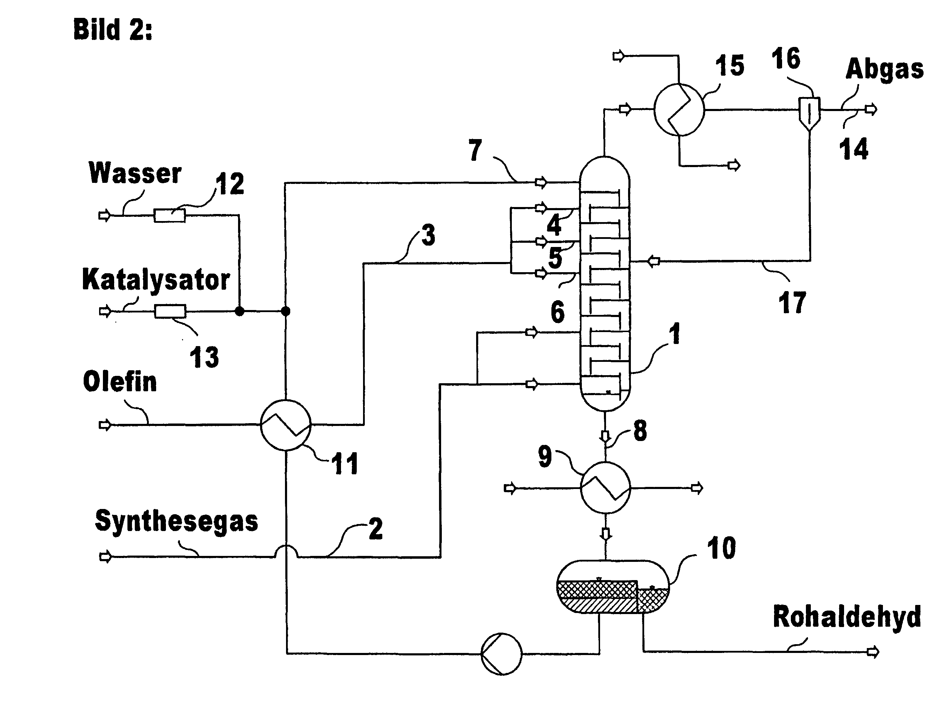

Die in Bild 2 dargestellte Verfahrensvariante unterscheidet sich vom Prozessablauf gemäß Bild 1 dadurch, dass das den Reaktor 1 über Leitung 14 verlassende Abgas in einem Wärmeaustauscher 15 abgekühlt wird. Es bildet sich ein Zweiphasensystem aus gasförmiger und flüssiger Phase. Beide Phasen werden in einem Abscheider 16 getrennt, der gasförmige Anteil wird aus dem System entfernt, der flüssige Anteil über eine Leitung 17 in die Reaktionskolonne 1 zurückgeführt. Die mit den Ziffern 2 bis 13 bezeichneten Anlageteile entsprechen (wie auch die Reaktionskolonne 1 und die Abgasleitung 14) denen des Bildes 1.

## Patentansprüche

1. Verfahren zur Hydroformylierung olefinisch ungesättigter Verbindungen in einem heterogenen Reaktionssystem unter Verwendung einer wässrigen Lösung als Katalysator, die Komplexverbindungen des Rhodiums mit wasserlöslichen organischen Phosphor(III)-verbindungen als Liganden und gegebenenfalls noch überschüssige wasserlösliche organische Phosphor(III)-verbindungen enthält, bei Drücken von 0,4 bis 10 MPa und Temperaturen von 50 bis 180°C, **dadurch gekennzeichnet, dass** die Umsetzung der Reaktionspartner in einer Reaktionskolonne erfolgt.

2. Verfahren nach Anspruch 1, **dadurch gekennzeichnet, dass** die Reaktionskolonne mit Einspeiseöffnungen für die Reaktionspartner und die Katalysatorlösung und mit Entnahmevorrichtungen für Produkt, Katalysatorfösung und Abgas versehen ist.

3. Verfahren nach Anspruch 1 oder 2, **dadurch gekennzeichnet, dass** die Reaktionskolonne eine Bodenkolonne, eine Packungskolonne oder eine Füllkörperkolonne ist.

4. Verfahren nach einem oder mehreren der Ansprüche 1 bis 3, **dadurch gekennzeichnet, dass** die Reaktionskolonne 5 bis 120, vorzugsweise 15 bis 40 Trennstufen besitzt.

5. Verfahren nach einem oder mehreren der Ansprüche 1 bis 4, **dadurch gekennzeichnet, dass** die Reaktionspartner olefinisch ungesättigte Verbindungen und Synthesegas und die Katalysatorlösung der Reaktionskolonne an getrennten Einspeisestellen zugeführt werden.

6. Verfahren nach einem oder mehreren der Ansprüche 1 bis 5, **dadurch gekennzeichnet, dass** die olefinisch ungesättigte Verbindung an einer oder an mehreren Einspeisestellen im oberen Bereich der Reaktionskolonne zugeführt wird.

7. Verfahren nach einem oder mehreren der Ansprüche 1 bis 6, **dadurch gekennzeichnet, dass** das Synthesegas an einer oder an mehreren Einspeisestellen im unteren Bereich, vorzugsweise am Fuß der Reaktionskolonne, zugeführt wird.

8. Verfahren nach einem oder mehreren der Ansprüche 1 bis 7, **dadurch gekennzeichnet, dass** die Zuführung der Katalysatorlösung im oberen Bereich der Reaktionskolonne, insbesondere auf die oberste Trennstufe der Reaktionskolonne, erfolgt.

9. Verfahren nach einem oder mehreren der Ansprüche 1 bis 5, **dadurch gekennzeichnet, dass** die Reaktionspartner olefinisch ungesättigte Verbindung und Synthesegas und die Katalysatorlösung jeweils an mehreren längs der Reaktionskolonne angeordneten Einspeisestellen zugeführt wird.

10. Verfahren nach einem oder mehreren der Ansprüche 1 bis 4 und 6 bis 8, **dadurch gekennzeichnet, dass** olefinisch ungesättigte Verbindung und Katalysator unterhalb des Kolonnenkopfes gemeinsam an einer oder mehreren Einspeisestellen der Reaktionskolonne zugeführt wird.

11. Verfahren nach einem oder mehreren der Ansprüche 1 bis 10, **dadurch gekennzeichnet, dass** die Reaktanten vor Einführung in die Reaktionskolonne vorgewärmt werden.

12. Verfahren nach Anspruch 11, **dadurch gekennzeichnet, dass** die Vorwärmung mit Prozessabwärme erfolgt.

13. Verfahren nach einem oder mehreren der Ansprüche 1 bis 12, **dadurch gekennzeichnet, dass** die Abführung der Reaktionswärme durch Vermittlung eines Hilfsmediums, wie Wasser oder Wasserdampf, erfolgt.

14. Verfahren nach einem oder mehreren der Ansprüche 1 bis 13, **dadurch gekennzeichnet, dass** das Abgas in eine gasförmige und eine flüssige Phase aufgetrennt und die flüssige Phase in die Reaktionskolonne zurückgeführt wird.

## Claims

1. A process for the hydroformylation of olefinically unsaturated compounds in a heterogeneous reaction system using a catalyst consisting of an aqueous solution comprising complexes of rhodium with water-soluble organic phosphorus(III) compounds as ligands and, if desired, excess water-soluble organic phosphorus(II1) compounds, at pressures of from 0.4 to 10 MPa and temperatures of from 50 to 180°C, wherein the reaction of the reactants is carried out in a reaction column.

2. The process as claimed in claim 1, wherein the reaction column is provided with feed openings for the reactants and the catalyst solution and with devices for taking off product, catalyst solution and offgas.

3. The process as claimed in claim 1 or 2, wherein the reaction column is a tray column, a column with ordered packing or a column with random packing elements.

4. The process as claimed in one or more of claims 1 to 3, wherein the reaction column has from 5 to 120, preferably from 15 to 40, separation stages.

5. The process as claimed in one or more of claims 1 to 4, wherein the reactants, namely olefinically unsaturated compounds and synthesis gas, and the catalyst solution are fed into the reaction column at separate feed points.

6. The process as claimed in one or more of claims 1 to 5, wherein the olefinically unsaturated compound is introduced in the upper region of the reaction column at one feed point or at a plurality of feed points.

7. The process as claimed in one or more of claims 1 to 6, wherein the synthesis gas is introduced in the lower region of the reaction column, preferably at the bottom of the reaction column, at one feed point or at a plurality of feed points.

8. The process as claimed in one or more of claims 1 to 7, wherein the catalyst solution is introduced in the upper region of the reaction column, in particular onto the uppermost separation stage of the reaction column.

9. The process as claimed in one or more of claims 1 to 5, wherein the reactants, namely olefinically unsaturated compound and synthesis gas, and the catalyst solution are each introduced at a plurality of feed points arranged along the reaction column.

10. The process as claimed in one or more of claims 1 to 4, and 6 to 8, wherein olefinically unsaturated compound and catalyst are introduced together below the top of the column at one or more feed points along the reaction column.

11. The process as claimed in one or more of claims 1 to 10, wherein the reactants are preheated before being introduced into the reaction column.

12. The process as claimed in claim 11, wherein preheating is carried out using heat evolved in the process

13. The process as claimed in one or more of claims 1 to 12, wherein the heat of reaction is removed by means of an auxiliary medium such as water or steam.

14. The process as claimed in one or more of claims 1 to 13, wherein the offgas is separated into a gaseous phase and a liquid phase and the liquid phase is returned to the reaction column.

## Revendications

1. Procédé pour l'hydroformylation de composés oléfiniquement insaturés dans un système de réaction hétérogène avec utilisation d'une solution aqueuse comme catalyseur, contenant des composés complexes du rhodium avec des composés organiques solubles dans l'eau du phosphore (III) comme ligands et, le cas échéant, des composés organiques solubles dans l'eau du phosphore (III) en excès, à des pressions de 0,4 à 10 MPa et des températures de 50 à 180°C, **caractérisé en ce que** la transformation des partenaires de réaction a lieu dans une colonne de réaction.

2. Procédé selon la revendication 1, **caractérisé en ce que** la colonne de réaction est pourvue d'alimentations pour les partenaires de réaction et la solution de catalyseur et de dispositifs de prélèvement pour le produit, la solution de catalyseur et les effluents gazeux.

3. Procédé selon la revendication 1 ou 2, **caractérisé en ce que** la colonne de réaction est une colonne à plateaux, une colonne à garniture ou une colonne à corps de remplissage.

4. Procédé selon l'une ou plusieurs des revendications 1 à 3, **caractérisé en ce que** la colonne de réaction présente 5 à 120, de préférence 15 à 40 étages de séparation.

5. Procédé selon l'une ou plusieurs des revendications 1 à 4, **caractérisé en ce que** les partenaires de réaction, les composés oléfiniquement insaturés et le gaz de synthèse et la solution de catalyseur sont introduits dans la colonne de réaction via des alimentations séparées.

6. Procédé selon l'une ou plusieurs des revendications 1 à 5, **caractérisé en ce que** le composé oléfiniquement insaturé est introduit dans la partie supérieure de la colonne de réaction via une ou plusieurs alimentations.

7. Procédé selon l'une ou plusieurs des revendications 1 à 6, **caractérisé en ce que** le gaz de synthèse est introduit dans la partie inférieure, de préférence au pied de la colonne de réaction via une ou plusieurs alimentations.

8. Procédé selon l'une ou plusieurs des revendications 1 à 7, **caractérisé en ce que** l'introduction de la solution de catalyseur est réalisée dans la partie supérieure de la colonne de réaction, en particulier sur l'étage de séparation supérieur de la colonne de réaction.

9. Procédé selon l'une ou plusieurs des revendications 1 à 5, **caractérisé en ce que** les partenaires de réaction, le composé oléfiniquement insaturé et le gaz de synthèse et la solution de catalyseur, sont à chaque fois introduit via plusieurs alimentations disposées le long de la colonne de réaction.

10. Procédé selon l'une ou plusieurs des revendications 1 à 4 et 6 à 8, **caractérisé en ce que** le composé oléfiniquement insaturé et le catalyseur sont introduits sous la tête de colonne, simultanément via une ou plusieurs alimentations dans la colonne de réaction.

11. Procédé selon l'une ou plusieurs des revendications 1 à 10, **caractérisé en ce que** les réactifs sont préchauffés avant l'introduction dans la colonne de réaction.

12. Procédé selon la revendication 11, **caractérisé en ce que** le préchauffage est réalisé avec de la chaleur provenant du processus.

13. Procédé selon l'une ou plusieurs des revendications 1 à 12, **caractérisé en ce que** l'évacuation de la chaleur de réaction est réalisée par l'intermédiaire d'un milieu auxiliaire, tel que l'eau ou la vapeur d'eau.

14. Procédé selon l'une ou plusieurs des revendications 1 à 13, **caractérisé en ce que** l'effluent gazeux est séparé en une phase gazeuse et une phase liquide et la phase liquide est recyclée dans la colonne de réaction.
